# EUROPEAN PATENT SPECIFICATION

(11) **EP 1 324 785 B1**
(45) Date of publication and mention of the grant of the patent: **28.04.2004**
(21) Application number: 01969935.4
(22) Date of filing: 19.09.2001
(51) Int. Cl.: A61L 29/08, A61L 29/16

(54) **CATHETER WITH HYDROPHILIC COATING COMPRISING AN ANTHRAQUINONE**
KATHETER MIT HYDROPHILER ANTHRACHINON-HALTIGER BESCHICHTUNG
CATHETER A REVETEMENT HYDROPHILE COMPRENANT UNE ANTHRAQUINONE

(30) Priority: 21.09.2000 GB 0023131
(43) Date of publication of application: 09.07.2003
(73) Proprietor: Hunter Urology Limited, Honiton, Devon EX14 4TP (GB)
(72) Inventor: HUNTER, Gary, Francis, Exeter, Devon EX2 8BP (GB)
(74) Representative: Craske, Stephen Allan
(86) International application number: PCT/GB2001/004171
(87) International publication number: WO 2002/024246

(56) References cited:
- WO-A-98/58990
- US-A- 4 539 234
- US-A- 5 772 640
- US-A- 5 848 995

## Description

### TECHNICAL FIELD OF THE INVENTION

This invention relates to catheters and particularly (without prejudice to the generality) urinary catheters.

### BACKGROUND

Catheterisation is common in adults and children with urinary retention or incomplete bladder emptying (known as post void residual - PVR). Urinary retention and PVR may occur if the bladder muscle (detrusor) has impaired contraction, the urethral sphincter does not open adequately (sphincter dysfunction), or both the detrusor and the sphincter do not function adequately (detrusor-sphincter dyssynergia).

A recognised technique for managing such conditions is to pass a small catheter into the bladder at regular intervals to remove urine which a patient is unable to void in the normal way. When the bladder has been drained the catheter is removed so that the patient is not constrained by a permanent in-dwelling catheter. The catheter may be inserted and removed by an assistant, but most patients can be taught a self-administration technique which is commonly known as intermittent self catheterisation (ISC), so that the patient is free to lead a more normal and active life.

Modern catheters are usually formed of a polymer such as polyvinyl chloride (PVC), polyamide (e.g. Nylon - RTM), latex rubber or silicone rubber. The external surface of the catheter is generally provided with a hydrophilic coating which gives a good hand grip when dry, but which becomes very slippery on contact with water so that the catheter can be inserted and withdrawn more easily resulting in less trauma to the user. The most widely used coatings comprise polyvinylpyrolidone. GB 1 600 963 discloses such a coating which comprises an interpolymer of polyvinylpyrolidone and polyurethane. EP 0 093 093 and EP 0 093 094 both disclose coatings formed by reacting an isocyanate compound with polyvinylpyrolidone. The coating can also include other constituents such as urea, sodium chloride or iodine (an antibacterial agent).

The present invention seeks to provide a new and inventive form of catheter.

### DETAILED DESCRIPTION OF THE INVENTION

The present invention proposes a catheter provided with an external hydrophilic surface coat comprising an anthraquinone.

Whilst the advantages of ISC are becoming more widely recognised the technique currently carries a substantial risk of urinary tract infection and damage to epithelial cell lining of the urethra, which may itself increase the risk of infection. By incorporating one or more anthraquinones these risks can be substantially reduced and can often be completely eliminated.

Anthraquinones possess many beneficial properties. They have strong bactericidal and antiviral properties, they fight fungal infections such as thrush, and they act as analgesic and anti-inflammatory agents. Preferred anthraquinones are aloin and emodin, which may to advantage be used together. Aloin is a constituent of the aloe vera plant, particularly aloe vera barbadensic(mylev), also known as aloe vera (linne), and aloe arborescens. Aloin and, in smaller quantities emodin, are extracted in a viscous yellow liquid obtained from the skin of the plant.

The hydrophilic coating preferably also comprises a saponin. These substances are surfactants which act to reduce surface tension and therefore enhance the hydrophilic properties of the catheter. Many are also powerfully antiseptic, acting to destroy bacteria, viruses, fungi and yeasts.

The surface coating preferably further includes an anti-inflammatory agent. Preferred anti-inflammatory agents comprise fatty acids.

A further preferred constituent of the surface coating is an antioxidant. Antioxidants help prevent injury to epithelial tissues and promote healing. Examples of preferred antioxidants are vitamins C and E, and beta-carotene. Selenium is another particularly useful antioxidant which protects against free radicals (molecules without an electron). Again, maximum benefit is achieved by including two or more antioxidants.

The surface coating preferably further comprises a mucopolysaccharide. Mucopolysaccharides help form a barrier against microbial invasion of the epithelium. A particularly preferred mucopolysaccharide is acemannan, a powerful stimulant of the immune system. Acemannan has been shown to stimulate macrophage activity causing them to produce immune agents such as interferon and interleukin.

The surface coating may, to advantage also comprise lignins, which penetrate the epithelium and render it permeable to other soluble ingredients of the surface coat.

The hydrophilic coating may include other substances which aid cell regeneration such as vitamins B1, B2, B3, B6 and B12, trace element minerals such as magnesium, manganese, zinc, copper, chromium, calcium, sodium, potassium, selenium, and essential Amino acids.

The coating may further comprise known hydrophilic coating polymers such as polyvinylpyrolidone which is deposited on the surface of the catheter together with the anthraquinone and other constituents in a known manner.

The catheter may be of a known form. Generally, the catheter will comprise a tube which has a closed, smoothly rounded tip at one end to aid insertion, with at least one lateral opening adjacent to the tip to allow urine to enter the tube when the tip enters the bladder. The tube may be formed of a range of polymers which include vinyl polymers such as polyvinyl chloride, latex or silicone rubbers, polyesters or polyacrylates.

It will be appreciated that the features disclosed herein may be present in any feasible combination. Whilst the above description lays emphasis on those areas which, in combination, are believed to be new, protection is claimed for any inventive combination of the features disclosed herein.

## Claims

1. A catheter provided with an external hydrophilic surface coat which includes an anthraquinone.

2. A catheter according to Claim 1, in which the anthraquinone is aloin.

3. A catheter according to Claim 1, in which the anthraquinone is emodin.

4. A catheter according to Claim 1, in which the hydrophilic surface coat includes a saponin.

5. A catheter according to Claim 1, in which the hydrophilic surface coat includes an anti-inflammatory agent.

6. A catheter according to Claim 5, in which the anti-inflammatory agent includes fatty acids.

7. A catheter according to Claim 1, in which the hydrophilic surface coat includes an antioxidant.

8. A catheter according to Claim 7, in which the antioxidant includes one or more of vitamin C, vitamin E, beta-carotene and selenium.

9. A catheter according to Claim 1, in which the hydrophilic surface coat includes a mucopolysaccharide.

10. A catheter according to Claim 1, in which the hydrophilic surface coat includes lignins.

## Patentansprüche

1. Ein Katheter ausgestattet mit einer externen hydrophilen Oberflächenbeschichtung, die ein Anthrachinon enthält.

2. Ein Katheter nach Anspruch 1, in dem das Anthrachinon Aloin ist.

3. Ein Katheter nach Anspruch 1, in dem das Anthrachinon Emodin ist.

4. Ein Katheter nach Anspruch 1, in dem die hydrophile Oberflächenbeschichtung ein Saponin enthält.

5. Ein Katheter nach Anspruch 1, in dem die hydrophile Oberflächenbeschichtung ein entzündungshemmendes Mittel enthält.

6. Ein Katheter nach Anspruch 5, in dem das entzündungshemmende Mittel Fettsäuren enthält.

7. Ein Katheter nach Anspruch 1, in dem die hydrophile Oberflächenbeschichtung ein Antioxidationsmittel enthält.

8. Ein Katheter nach Anspruch 7, in dem das Antioxidationsmittel einen oder mehrere der Stoffe Vitamin C, Vitamin E, Betacarotin und Selen enthält.

9. Ein Katheter nach Anspruch 1, in dem die hydrophile Oberflächenbeschichtung ein Mucopolysaccharid enthält.

10. Ein Katheter nach Anspruch 1, in dem die hydrophile Oberflächenbeschichtung Lignine enthält.

## Revendications

1. Cathéter muni d'un revêtement de surface hydrophile externe qui comprend une anthraquinone.

2. Cathéter selon la revendication 1, dans lequel l'anthraquinone est l'aloïne.

3. Cathéter selon la revendication 1, dans lequel l'anthraquinone est l'émodine.

4. Cathéter selon la revendication 1, dans lequel le revêtement de surface hydrophile comprend une saponine.

5. Cathéter selon la revendication 1, dans lequel le revêtement de surface hydrophile comprend un agent anti-inflammatoire.

6. Cathéter selon la revendication 5, dans lequel l'agent anti-inflammatoire comprend des acides gras.

7. Cathéter selon la revendication 1, dans lequel le revêtement de surface hydrophile comprend un anti-oxydant.

8. Cathéter selon la revendication 7, dans lequel l'anti-oxydant comprend une ou plusieurs parmi la vitamine C, la vitamine E, le bêta-carotène et le sélénium.

9. Cathéter selon la revendication 1, dans lequel le revêtement de surface hydrophile comprend un mucopolysaccharide.

10. Cathéter selon la revendication 1, dans lequel le revêtement de surface hydrophile comprend des lignines.
